# EUROPEAN PATENT SPECIFICATION

(11) **EP 1 920 258 B1**
(45) Date of publication and mention of the grant of the patent: **11.12.2013**
(21) Application number: 06788794.3
(22) Date of filing: 27.07.2006
(51) Int. Cl.: G01N 33/68

(54) **METHODS AFFECTING MARKERS IN PATIENTS HAVING VASCULAR DISEASE**
VERFAHREN MIT AUSWIRKUNG AUF MARKER IN PATIENTEN MIT GEFÄSSLEIDEN
PROCEDES DESTINES A MODIFIER DES MARQUEURS CHEZ DES PATIENTS ATTEINTS D'UNE MALADIE VASCULAIRE

(30) Priority: 27.07.2005 US 190049
(43) Date of publication of application: 14.05.2008
(73) Proprietor: Covidien LP, Mansfield, MA 02048 (US)
(72) Inventor: SIMPSON, John, B., Woodside, California 94062 (US)
(74) Representative: Elsy, David
(86) International application number: PCT/US2006/029415
(87) International publication number: WO 2007/014330

(56) References cited:
- EP-A1- 0 999 447
- WO-A-03/016910
- WO-A2-2004/089184
- HOJO Y ET AL: "Interleukin 6 expression in coronary circulation after coronary angioplasty as a risk factor for restenosis" HEART (LONDON), vol. 84, no. 1, July 2000 (2000-07), pages 83-87, XP002404363 ISSN: 1355-6037
- TASHIRO HIDEKI ET AL: "Role of cytokines in the pathogenesis of restenosis after percutaneous transluminal coronary angioplasty" CORONARY ARTERY DISEASE, vol. 12, no. 2, March 2001 (2001-03), pages 107-113, XP008070422 ISSN: 0954-6928
- HOJO YUKIHIRO ET AL: "Chemokine expression in coronary circulation after coronary angioplasty as a prognostic factor for restenosis" ATHEROSCLEROSIS, vol. 156, no. 1, May 2001 (2001-05), pages 165-170, XP002404364 ISSN: 0021-9150
- KURZ ROBERT W ET AL: "Increased serum concentrations of adhesion molecules after coronary angioplasty" CLINICAL SCIENCE (LONDON), vol. 87, no. 6, 1994, pages 627-633, XP008070420 ISSN: 0143-5221
- BRILAKIS EMMANOUIL S ET AL: "Association of lipoprotein-associated phospholipase A2 levels with coronary artery disease risk factors, angiographic coronary artery disease, and major adverse events at follow-up" EUROPEAN HEART JOURNAL, vol. 26, no. 2, January 2005 (2005-01), pages 137-144, XP002404365 ISSN: 0195-668X
- BALLANTYNE CHRISTIE M ET AL: "Lipoprotein-associated phospholipase A2, high-sensitivity C-reactive protein, and risk for incident coronary heart disease in middle-aged men and women in the Atherosclerosis Risk in Communities (ARIC) study." CIRCULATION, vol. 109, no. 7, 24 February 2004 (2004-02-24), pages 837-842, XP002404366 ISSN: 0009-7322
- HOJO YUKIHIRO ET AL: "Matrix metalloproteinase expression in the coronary circulation induced by coronary angioplasty" ATHEROSCLEROSIS, vol. 161, no. 1, March 2002 (2002-03), pages 185-192, XP002404367 ISSN: 0021-9150
- CIPOLLONE F ET AL: "High preprocedural non-HDL cholesterol is associated with enhanced oxidative stress and monocyte activation after coronary angioplasty: Possible implications in restenosis." HEART (LONDON), vol. 89, no. 7, July 2003 (2003-07), pages 773-779, XP002404368 ISSN: 1355-6037
- WINKLER KARL ET AL: "Platelet-activating factor acetylhydrolase activity indicates angiographic coronary artery disease independently of systemic inflammation and other risk factors - The Ludwigshafen risk and cardiovascular health study" CIRCULATION, vol. 111, no. 8, 1 March 2005 (2005-03-01), pages 980-987, XP002404369 ISSN: 0009-7322
- MUKHERJEE D ET AL: "Elective coronary revascularization, an iatrogenic form of acute coronary syndrome: How can clinicians reduce the risks?" AMERICAN HEART JOURNAL, MOSBY- YEAR BOOK INC., ST. LOUIS, MO, US, vol. 148, no. 3, September 2004 (2004-09), pages 371-377, XP004573132 ISSN: 0002-8703
- INOUE T ET AL: "EXPRESSION OF POLYMORPHONUCLEAR LEUKOCYTE ADHESION MOLECULES AND ITS CLINICAL SIGNIFICANCE IN PATIENTS TREATED WITH PERCUTANEOUS TRANSLUMINAL CORONARY ANGIOPLASTY" JOURNAL OF THE AMERICAN COLLEGE OF CARDIOLOGY, vol. 28, no. 5, 1 November 1996 (1996-11-01), pages 1127-1133, XP002911565 ISSN: 0735-1097
- HORIE HAJIME ET AL: "Association of an acute reduction in lipoprotein(a) with coronary artery restenosis after percutaneous transluminal coronary angioplasty" CIRCULATION, vol. 96, no. 1, 1997, pages 166-173, XP008070419 ISSN: 0009-7322
- BALLANTYNE CHRISTIE M ET AL: "Markers of inflammation and their clinical significance" ATHEROSCLEROSIS SUPPLEMENTS, vol. 6, no. 2, May 2005 (2005-05), pages 21-29, XP002404370 ISSN: 1567-5688
- FRANÇOIS MACH: 'INFLAMMATION IS A CRUCIAL FEATURE OF ATHEROSCLEROSIS AND A POTENTIAL TARGET TO REDUCE CARDIOVASCULAR EVENTS' HANDB EXP PHARMACOL. 01 April 2005, pages 697 - 722, XP055045488
- HANSSON GORAN K ET AL: "Inflammation and atherosclerosis", ANNUAL REVIEW OF PATHOLOGY: MECHANISMS OF DISEASE, ANNUAL REVIEWS, US, vol. 1, no. 1, 1 January 2006 (2006-01-01) , pages 297-329, XP009145346, ISSN: 1553-4006
- BALLANTYNE C M ET AL: "Markers of inflammation and their clinical significance", ATHEROSCLEROSIS SUPPLEMENTS, ELSEVIER, AMSTERDAM, NL, vol. 6, no. 2, 1 May 2005 (2005-05-01), pages 21-29, XP027685487, ISSN: 1567-5688 [retrieved on 2005-05-01]
- ZERNECKE A ET AL: "Inflammatory mediators in atherosclerotic vascular disease", BASIC RESEARCH IN CARDIOLOGY, STEINKOPFF-VERLAG, DA, vol. 100, no. 2, 1 March 2005 (2005-03-01) , pages 93-101, XP019383130, ISSN: 1435-1803, DOI: 10.1007/S00395-005-0511-6
- BRAUN LYNNE T ET AL: "Lp-PLA2: A new target for statin therapy.", CURRENT ATHEROSCLEROSIS REPORTS JAN 2010, vol. 12, no. 1, January 2010 (2010-01), pages 29-33, ISSN: 1534-6242

## Description

### TECHNICAL FIELD

The present invention relates to an ex vivo method of monitoring the success of atherectomy.

### BACKGROUND ART

The accumulation of atheromatous tissue on the inner walls of vascular lumens, particularly arterial lumens of the coronary and peripheral vasculature, results in a condition known as vascular disease. Vascular disease occurs naturally as a result of aging, but may also be aggravated by factors such as poor diet, hypertension, heredity, vascular injury, and the like. Atheromatous and other vascular deposits restrict blood flow and can cause ischemia which, in acute cases, can result in myocardial infarction.

Atheromatous tissue can have widely varying properties, with some deposits being relatively soft, some fibrous, some calcified and some a combination of soft, fibrous, and calcified. Calcified deposits are frequently referred to as plaque. A special type of plaque is called vulnerable plaque which has high lipid content and some soft or fibrous tissue as well. Vulnerable plaque has a propensity to break off and cause cardiac or other infarctions with little or no warning.

Vascular disease and a related condition, restenosis (which is the re-filling of the vasculature with atheromatous tissue after an initial removal) can be treated in a variety of ways, including drugs, bypass surgery, and a variety of catheter-based approaches which rely on intravascular debulking or removal of the atheromatous or other tissue occluding a blood vessel. Using older atherectomy devices and procedures, the goal was merely to remove enough tissue to open an occlusion, i.e., to clear enough tissue from the lumen so that blood flow could resume, at least for a time. Newer devices can remove more tissue.

There is a need in the art for additional methods of treating patients having vascular disease. More particularly, there is a need in the art for methods which monitor and result in the improvement of the blood chemistry and physiology of patients.

### DISCLOSURE OF THE INVENTION

The invention provides an ex vivo method of monitoring the success of atherectomy, the atherectomy comprising removal of diseased vascular tissue from a vascular lumen of a patient, wherein the method comprises testing a sample obtained from a patient prior to atherectomy and testing a sample obtained from the patient after atherectomy for the level of a marker, wherein a decrease in the level of a marker selected from C reactive protein (CRP), PDGF, PDGF receptor, FGF, VEGF, VCAM-1 and IL-6 indicates success of the atherectomy.

### BRIEF DESCRIPTION OF THE DRAWINGS

TABLE. 1 is a list exemplary markers which can be determined as nucleic acid (mRNA or cDNA) or as protein.

### DETAILED DESCRIPTION OF THE INVENTION

### Introduction

It is a discovery of the present inventor that removal of vascular tissue in an atherectomy procedure improves the patient's overall health, including physiology, blood chemistry, and/or markers of disease. Such global effects can be used to monitor success of atherectomy as well as to monitor future progression of disease and to plan future treatments.

### Modulating a Presence or Absence of a Marker

OIL If enough vascular tissue is removed from the patient, the marker level (including its presence or absence) is modulated (i.e., the level of the marker in the patient increases or decreases). Either an increase in a beneficial marker or a decrease in a detrimental marker indicates an improvement in the patient as a result of the atherectomy procedure upon removal of sufficient vascular tissue from the patient.

A patient to be treated will generally have some form of vascular disease, or a disease associated with vascular disease, where the condition results in an accumulation of material in the vasculature of the patient. Vascular disease results from an accumulation of material in vascular lumens. The accumulated material is referred to as vascular tissue.

A marker can be determined in the patient. The marker may be found in blood, lymph, serum, tears, saliva, urine, stool, sputum, or tissue of the patient. The tissue can be vascular tissue or non-vascular tissue. The marker is selected for its association with the vascular disease or some aspect of the disease. Thus the marker can be expressed and present in healthy patients, and characteristically absent in patients having vascular disease. Alternatively, the marker can be absent in healthy patients and elevated in patients having vascular disease. The markers are typically differentially expressed or found in healthy versus vascular disease patients. To determine level (including presence or absence) of a particular marker, standard assays for that marker can be used. As the marker may be present, for example, in vascular tissue, non-vascular tissue, blood, or lymph, the assay for the marker may be tailored to the source of the marker. The assay to determine the marker will be tailored to the nature of the marker, *e.g., a.* cell, a protein, a polypeptide, an expression product of DNA, an RNA, or other marker. Standard assays known in the art can be used to identify any of the markers for vascular disease.

The marker is defmed in claim 1.

The vascular tissue can be removed from the body by atherectomy procedure, using such devices as those described herein. Any atherectomy device can be used, providing the device is able to remove sufficient vascular tissue to effect a change in the marker that represents a change in the physiology or body chemistry of the patient.

The vascular tissue removed can be any tissue found in the vasculature, including such tissue as, for example, arterial plaque, vulnerable plaque, inflamed tissue, arterial tissue, calcified tissue, thrombotic tissue, lipid-rich tissue, foam cell tissue, macrophage-rich tissue, hypocellular tissue, fibrotic tissue, hypercellular tissue, and diseased tissue. Vascular tissue can be soft and fibrous, calcified, and lipid. The specific components of a patient's vascular tissue will typically vary from patient to patient and may indicate the relative seriousness in the patient's condition. For example, plaque or calcified vascular tissue may severely restrict flow and should be removed. Vulnerable plaque contains lipids and other vessel wall components, and may be responsible for cardiac infarctions that occur in otherwise asymptomatic patients. Vulnerable plaque should be removed, when possible. The tissue removed from the vessel wall may also comprise inflamed tissue.

The vascular tissue can be removed from any part of the patient's vasculature that is accessible using an atherectomy device. Thus, the vascular tissue can be removed from, for example, a vessel such as a blood vessel, a peripheral artery, a coronary artery and a carotid artery.

In general, the targeted vessel should be identified as having vascular tissue prior to the atherectomy procedure (for example, by a visualization technique such as sonography). Vascular tissue is then removed from the targeted vessel by a tool or catheter capable of removing sufficient quantities of vascular tissue.

A sufficient quantity of tissue is that amount that is a therapeutically significant portion of Vascular tissue sufficient to modulate the level of the marker. The modulated level could be, for example, the level within what would be considered a healthy range, or close to the level of what is a healthy range. What constitutes a sufficient quantity of tissue may vary from patient to patient, but can be estimated. Particularly, a sufficient quantity of tissue can be determined from a study of a population. For example, where it is determined that removal of two grams or more of vascular tissue is sufficient in most members of the population to modulate the marker levels to close to the normal range for a healthy adult, removal of two grams or more of tissue would be considered sufficient. In some patients, however, removal of less could be sufficient. In other patients, more could be required.

The level of a beneficial marker increases as a result of removal of sufficient vascular tissue, and the level of a detrimental marker decreases as a result of removal of sufficient vascular tissue. It should be noted that the amount of the change in the marker level (either up or down) is a significant amount. Typically the change is enough to indicate that the patient's body chemistry has been altered for the better, at least as represented by the change in the marker level. A significant change in a marker level returns the marker level to the range of what it would be in a healthy person, or at least substantially closer to that range than it was previously.

The process of measuring or determining the marker before vascular tissue removal, can be optionally repeated in a re-testing of the marker level after vascular tissue removal. Even if it is believed that so much vascular tissue has been removed that a modulation of a marker level in the patient has been effected, a second test of the marker level after the tissue removal can be made. This second test can indicate whether the vascular tissue removal process should be repeated, and can be used to monitor disease and/or accumulation of vascular tissue. Post-procedure monitoring can be used to determine treatments to reduce risk of cardiac failure or other negative outcomes of acute and moderate vascular disease. Changes in the patient physiology or blood chemistry can be measured by observing modulation of the presence, absence, or level of a marker in the patient's blood, tissue or lymph, or by a change that effects a modification of the marker itself (e.g., a conformational change in the marker).

The quantity of tissue removed by the method can be any amount, but is typically from about 1 mg to about 2000 mg. Typically the amount of tissue is about 1 mg to about 100 mg, about 10 mg to about 1000 mg, about 50 mg to about 500 mg, about 100 mg to about 400 mg, 400 mg to about 500 mg, 500 mg to about 600 mg, about 600 mg to about 700 mg, 700 mg to about 800 mg, or about 800 mg to about 2000 mg. In a typical procedure about 400 mg to about 600 mg of tissue is removed. Removed tissue may optionally be tested and/or archived.

Measurement of a level of the marker of interest after removal of Vascular tissue can be made to determine that sufficient tissue, particularly tissue comprising atherosclerotic plaque, has indeed been removed. The confirmatory measurement should be made in the same way that the first baseline measurement (prior to the removal procedure) has been made in order to establish a valid comparison. If it is determined that sufficient plaque has not been removed in the first procedure more vascular tissue can be removed and the marker level can be measured again. What will constitute a sufficient modulation in the marker level will depend on a number of factors, including but not limited to the character of the marker itself, the normal range for that marker in a population of individuals, and the level of the marker in the unhealthy patient before tissue removal. Generally, removing a therapeutically significant portion of vascular tissue from a patient will result in sufficient modulation of the level of the marker, i.e., a significant increase or decrease in a marker level or change in the presence or absence of the marker, particularly where the marker of interest is closely associated with overall cardiac and/or vascular health. For some markers, the desired modulation will be a decrease in the level of the marker in the patient. For other markers, it will be an increase in the level of the marker. In some cases, due to the time lag between removal of Vascular tissue and the body's response to that change, it may take more than a few hours or a day or a few days to determine that the marker or its level has indeed been modulated or modified in response to the removal of vascular tissue. Generally, however, a day or a few days will be sufficient waiting time before measuring the marker level to determine that sufficient or a therapeutically significant portion of vascular tissue has been removed. In most cases, the second measurement of the marker or its level can be made within one week after removal of vascular tissue.

Modulating the level of a marker includes a quantitative change in the amount of the marker, as well as a change from presence to absence or absence to presence. The marker level may be measured a second or more times after the vascular tissue is removed, in order to confirm whether sufficient vascular tissue has been removed. The desired target modulation can be, for example, modulation of the marker level to what it would be in normal healthy individuals showing no symptoms of vascular disease. The measurement can be taken immediately after vascular tissue removal, or some other period of time thereafter, such as within one minute, within one hour, within one day, within one week, within one month, within three months, within six months, within nine months, within one year, within two years, within three years, within four years, within five years, or within ten years. This period of time can be preselected as part of an overall protocol of treatment and follow-up.

The marker selected for observation and modulation can be any marker, whether from blood lymph or tissue or any other part of the body that relates meaningfully to monitoring and treating a person having vascular disease. The marker for inflammation can comprise C reactive protein (CRP). Some specific markers that can be the target of modulation or modification though not of the invention are listed in Fig. 1.

### Atherectomy Catheters

Modulating a presence or absence of a marker can be accomplished using an atherectomy catheter that removes tissue from a vascular lumen of a patient. An atherectomy catheter is introduced percutaneously into a patient and the catheter is directed to a site in the vascular lumen containing tissue. Often the location of the tissue for removal can be identified by a visualization technique such as sonography. Removing sufficient vascular tissue can involve using a catheter capable of removing large amounts of tissue from the vasculature, in single or multiple passes at a site of partial or total occlusion in the vasculature. The elements of such atherectomy devices can include, for example, a rotating cutter, a collection chamber, a cutting window, and that the catheter can advance through the material in a site while cutting and collecting the tissue. Removal of sufficient vascular tissue from a patient will typically require accessing and removing tissue from more than one site in the patient. The second or subsequent sites can be in the same lumen as the removal of the first aliquot of tissue, or may be in a different lumen. What tissue, and from where in the patient it is removed will tend to be a patient specific determination, depending on where the patient's vascular tissue is located, and how much of it there is. The following are more details on some atherectomy catheters that are capable of removing sufficient tissue from the vascular lumens of patients having vascular disease.

The methods modulating the presence or absence of a marker can involve introducing a percutanous catheter in the patient and directing the catheter to a site in a vascular lumen containing tissue. Sufficient vascular tissue is then removed from said patient to modulate a presence or absence of the marker. Removing sufficient vascular tissue can comprise the following steps: providing a catheter having a rotating cutter, a collection chamber, and a cutting window, the rotating cutter being movable between a stored position and an exposed position, at least part of the rotating cutter becoming exposed through the cutting window when moving to the exposed position; exposing the cutter by moving the cutter to the exposed position; advancing the catheter to move the rotating cutter through material in a site in the body lumen, the rotating cutter remaining in the exposed position so that the cutter and the window maintain their orientation with respect to one another when advancing the catheter through the material, the material cut by the rotating cutter comprising vascular tissue and being directed through the cutting window and into the collection chamber as the catheter is advanced through the material, and removing the material from the collection chamber.

The technique may comprise prior to removing the material from the collection chamber, moving the cutter to the stored position, repositioning the catheter at a second site, exposing the cutter by moving the cutter to the exposed position, advancing the catheter to move the rotating cutter through material in the second site, the rotating cutter remaining in the exposed position so that the cutter and the window maintain their orientation with respect to one another when advancing the catheter through the material, the material cut by the rotating cutter being directed through the cutting window and into the collection chamber as the catheter is advanced through the material.

The catheters and methods are designed to debulk atheroma and other occlusive material from diseased body lumens, and in particular coronary arteries, *de novo* lesions, and in-stent restenosis lesions. The catheters and methods, however, are also suitable for treating stenoses of body lumens and other hyperplastic and neoplastic conditions in other body lumens, such as the ureter, the biliary duct, respiratory passages, the pancreatic duct, the lynphatic duct, and the like. Neoplastic cell growth will often occur as a result of a tumor surrounding and intruding into a body lumen. Debulking of such material can thus be beneficial to maintain patency of the body lumen and can alter body chemistry or physiology as indicated by a modulation of a presence or absence of a marker in the patient. While the remaining discussion is directed at debulking and passing through atheromatous or thrombotic occlusive material in vasculature, it will be appreciated that the systems and methods of the present invention can be used to remove a variety of occlusive, stenotic, or hyperplastic material in a variety of body lumens.

Apparatus will generally comprise catheters having catheter bodies adapted for intraluminal introduction to the target body lumen. The dimensions and other physical characteristics of the catheter bodies will vary significantly depending on the body lumen which is to be accessed. In the exemplary case of atherectomy catheters intended for intravascular introduction, the proximal portions of the catheter bodies will typically be very flexible and suitable for introduction over a guidewire to a target site within the vasculature. In particular, catheters can be intended for "over-the-wire" introduction when a guidewire channel extends fully through the catheter body or for "rapid exchange" introduction where the guidewire channel extends only through a distal portion of the catheter body. In other cases, it may be possible to provide a fixed or integral coil tip or guidewire tip on the distal portion of the catheter or even dispense with the guidewire entirely. For convenience of illustration, guidewires will not be shown in all embodiments, but it should be appreciated that they can be incorporated into any of these embodiments.

Catheter bodies intended for intravascular introduction will typically have a length in the range from 50 cm to 200 cm and an outer diameter in the range from 1 French to 12 French (0.33 mm: 1 French), usually from 3 French to 9 French. In the case of coronary catheters, the length is typically in the range from 125 cm to 200 cm, the diameter is preferably below 8 French, more preferably below 7 French, and most preferably in the range from 2 French to 7 French. Catheter bodies will typically be composed of an organic polymer which is fabricated by conventional extrusion techniques. Suitable polymers include polyvinylchloride, polyurethanes, polyesters, polytetrafluoroethylenes (PTFE), silicone rubbers, natural rubbers, and the like. Optionally, the catheter body may be reinforced with braid, helical wires, coils, axial filaments, or the like, in order to increase rotational strength, column strength, toughness, push ability, and the like. Suitable catheter bodies may be formed by extrusion, with one or more channels being provided when desired. The catheter diameter can be modified by heat expansion and shrinkage using conventional techniques. The resulting catheters will thus be suitable for introduction to the vascular system, often the coronary arteries, by conventional techniques.

The distal portion of the catheters may have a wide variety of forms and structures. In many embodiments, a distal portion of the catheter is more rigid than a proximal portion, but in other embodiments the distal portion may be equally as flexible as the proximal portion. One aspect provides catheters having a distal portion with a reduced rigid length. The reduced rigid length can allow the catheters to access and treat tortuous vessels and small diameter body lumens. In most embodiments a rigid distal portion or housing of the catheter body will have a diameter that generally matches the proximal portion of the catheter body, however, in other embodiments, the distal portion may be larger or smaller than the flexible portion of the catheter.

A rigid distal portion of a catheter body can be formed from materials which are rigid or which have very low flexibilities, such as metals, hard plastics, composite materials, NiTi, steel with a coating such as titanium nitride, tantalum, ME-92®, diamonds, or the like. Most usually, the distal end of the catheter body will be formed from stainless steel or platinum/iridium. The length of the rigid distal portion may vary widely, typically being in the range from 5 mm to 35 mm, more usually from 10 mm to 25 mm, and preferably between 6 mm and 8 mm. In contrast, conventional catheters typically have rigid lengths of approximately 16 mm.

The side opening windows will typically have a length of approximately 2 mm. In other embodiments, however, the side opening cutting window can be larger or smaller, but should be large enough to allow the cutter to protrude a predetermined distance that is sufficient to debulk material from the body lumen.

The catheters can include a flexible atraumatic distal tip coupled to the rigid distal portion of the catheter. For example, an integrated distal tip can increase the safety of the catheter by eliminating the joint between the distal tip and the catheter body. The integral tip can. provide a smoother inner diameter for ease of tissue movement into a collection chamber in the tip. During manufacturing, the transition from the housing to the flexible distal tip can be finished with a polymer laminate over the material housing. No weld, crimp, or screw joint is usually required.

The atraumatic distal tip permits advancing the catheter distally through the blood vessel or other body lumen while reducing any damage caused to the body lumen by the catheter. Typically, the distal tip will have a guidewire channel to permit the catheter to be guided to the target lesion over a guidewire. In some exemplary configurations, the atraumatic distal tip comprises a coil. In some configurations the. distal tip has a rounded, blunt distal end. The catheter body can be tubular and have a forward-facing circular aperture which communicates with the atraumatic tip. A collection chamber can be housed within the distal tip to store material removed from the body lumen. The combination of the rigid distal end and the flexible distal tip is approximately 30 mm.

A rotatable cutter or other tissue debulking assembly may be disposed in the distal portion of the catheter to sever material which is adjacent to or received within the cutting window. In an exemplary embodiment, the cutter is movably disposed in the distal portion of the catheter body and movable across a side opening window. A straight or serrated cutting blade or other element can be formed integrally along a distal or proximal edge of the cutting window to assist in severing material from the body lumen. In one particular embodiment, the cutter has a diameter of approximately 1.14 mm. It should be appreciated however, that the diameter of the cutter will depend primarily on the diameter of the distal portion of the catheter body.

In exemplary embodiments, activation of an input device can deflect a distal portion of the catheter relative to the proximal portion of the catheter. Angular deflection of the distal portion may serve one or more purposes in various embodiments. Generally, for example, deflection of the distal portion increases the effective "diameter" of the catheter and causes the debulking assembly to be urged against material in a lumen, such as atherosclerotic plaque. In other embodiments, deflection of the distal portion may act to expose a debulking assembly through a window for contacting material in a lumen. In some embodiments, for example, activation of the input device moves the debulking assembly over a ramp or cam so that a portion of the rigid distal portion and flexible tip are caused to drop out of the path of the debulking assembly so as to expose the debulking assembly through the window. In some embodiments, deflection may both urge a portion of the catheter into material in a lumen and expose a tissue debulking assembly.

Movement of a tissue debulking assembly may cause deflection of a portion of the catheter or that deflection of the catheter may cause movement or exposure of a tissue debulking assembly, in various embodiments. In other embodiments, deflection of a portion of the catheter and movement of the tissue debulking assembly may be causally unconnected events. Any suitable combination of deflecting, exposing of a debulking assembly and the like is contemplated. In carrying out deflection, exposure and/or the like, a single input device may be used, so that a user may, for example, deflect a portion of a catheter and expose a tissue debulking assembly using a single input device operable by one hand. In other embodiments, rotation of a tissue debulking assembly may also be activated by the same, single input device. In other embodiments, multiple input devices may be used.

Some embodiments further help to urge the debulking assembly intro contact with target tissue by including a proximal portion of the catheter body having a rigid, shaped or defonnable portion that better contacts the vascular tissue. For example, some embodiments include a proximal portion with a bend that urges the debulking assembly toward a side of the lumen to be debulked. In other embodiments, one side of the proximal portion is less rigid than the other side. Thus, when tension is placed on the catheter in a proximal direction (as when pulling the debulking assembly proximally for use), one side of the proximal portion collapses more than the other, causing the catheter body to bend and the debulking assembly to move toward a side of the lumen to be debulked.

In exemplary embodiments, the debulking assembly comprises a rotatable cutter that is movable outside the window. By moving the cutter outside of the cutting window beyond an outer diameter of the distal portion of the catheter, the cutter is able to contact and sever material that does not invaginate into the cutting window. In a specific configuration, the rotating cutter can be moved over the cam within the rigid, or distal, portion of the catheter body so that the cutting edge is moved out of the window. Moving the rotating cutter outside of the cutting window and advancing the entire catheter body distally, a large amount of occlusive material can be removed. Consequently, the amount of material that can be removed is not limited by the size of the cutting window.

Certain embodiments provide methods for *in vivo* excising and removing material from the inner wall of one or more lumen that is of higher quantity and quality than prior devices or methods. The material removed therefore is better suited for use in various testing methods. Particularly, the methods provide sufficient material or better quality and quantity for use in one or more tests from a single percutaneous translumenal lumenectomy procedure. Further, the material typically maintains the structure possessed by the material *in vivo*. This provides for the ability to carry out certain tests, such as histology, cytopathology, and other tests that have been difficult to perform using prior devices and methods.

In one embodiment the method for excising and testing material from a body lumen comprises the steps of providing a catheter having a rotating cutter, a collection chamber, and a cutting window, the rotating cutter being movable between a stored position and an exposed position, at least part of the rotating cutter becoming exposed through the cutting widow when moving to the exposed position. The catheter is advanced transluminally through the body lumen to move or plane the rotating cutter through material in a first site in the body lumen, the rotating cutter remaining in the exposed position so that the cutter and the widow maintain their orientation with respect to one another when advancing the catheter and planing through the material. The planing action of the present invention provides a substantially consistent and even tissue removal through the body lumen. The contiguous strand of material cut by the rotating cutter is directed through the cutting widow and into the collection chamber as the catheter is advanced through the material. The material can then be removed from the collection chamber and one or more tests performed on at least a portion of the material removed from the collection chamber.

The material excised from the body lumen will vary in length and will depend on the catheter configuration, the type of material removed, the body lumen, and the like. However, in certain embodiments, the material will be in the form of strands that have a substantially consistent depth and width of tissue cuts. The material is typically longer than the length of the cutting window (but it may be shorter), and typically has a length of about 2.0 mm or longer, and sometimes between about 0.5 cm up to about 10 cm or longer in length. Advantageously, the planing action of the catheter provides a material tissue structure that reflects the actual *in vivo* tissue structure, and provides information about larger portions of the disease state of the body lumen.

In another embodiment the method can further comprise i) moving the cutter to the stored position, ii) repositioning the catheter at a second site, iii) exposing the cutter by moving the cutter to the exposed position, and iv) advancing the catheter to move the rotating cutter through material in the second site, the rotating cutter remaining in the exposed position so that the cutter and the widow maintain their orientation with respect to one another when advancing the catheter through the material, the material cut by the rotating cutter being directed through the cutting widow and into the collection chamber as the catheter is advanced through the material. The first and second sites can be in either the same or a different body lumen.

Another embodiment for removing material from a vascular location comprises the steps of providing a catheter having a body, an opening leading to a collection chamber, and a cutter, the cutter being movable between a stored position and an exposed position, the cutter becoming at least partially exposed when moving from the stored position to the exposed position. The catheter is then percutaneously introduced into and transluminal^ advanced through a patient's vascular system with the cutter in the stored position, the catheter being introduced into the vascular location where material is to be removed. The cutter is then exposed by moving the cutter to the exposed position and the cutter is rotated. The catheter is then advanced after the exposing step and during the rotating step, wherein the rotating cutter and the opening advance together so that material cut by the rotating cutter is directed through the opening and into the collection chamber as the catheter is advanced. Subsequent to excising the material the catheter is removed from the vascular location and the material collected in the collection chamber is harvested and one or more tests on at least a portion of the material removed from the collection chamber can be carried out.

The material removed from the collection chamber, or a portion thereof, can be placed in a preserving agent, a tissue fixative, and or a preparation agent suitable for a desired test prior to testing the material. The material removed from the patient by this method is typically at least one or more strip(s) of material that maintains the structure of the material *in vivo.* The quantity of material removed by the method can be from about 1 mg to about 2000 mg. Typically the amount of material is about 1 mg to about 100 mg, about 100 mg to about 200 mg, about 200 mg to about 300 mg, 300 mg to about 400 mg, 400 mg to about 500 mg, 500 mg to about 600 mg, about 600 mg to about 700 mg, 700 mg to about 800 mg, or about 800 mg to about 2000 mg. In a typical procedure about 400 mg to about 600 mg of material is removed and available for testing and/or storage. A preferred embodiment provides for the collection of one or more strips of material from the inner surface of the lumen that is longer than a largest dimension of the cutting window. In a particular example, the material can comprise plaque tissue. The material can be collected from a single site or at least one additional site in the same or a different body lumen.

As will be described in detail below, in some situations it is preferable to provide a serrated cutting edge, while in other situations it may be preferable to provide a smooth cutting edge. Optionally, the cutting edge of either or both the blades may be hardened, e.g., by application of a coating. A preferred coating material is a chromium based material, available from ME-92, Inc., which may be applied according to manufacturer's instructions. In some embodiments, the cutter includes a tungsten carbide cutting edge. Other rotatable and axially movable cutting blades are described in U.S. Patent Nos. 5,674,232; 5,242,460; 5,312,425; 5,431,673; and 4,771,774. In some embodiments, a rotatable cutter includes a beveled edge for removal of material from a body lumen while preventing injury to the lumen. In still other embodiments, a tissue debulking assembly may include alternative or additional features for debulking a lumen. For example, the debulking assembly may include, but is not limited to, a radio frequency device, an abrasion device, a laser cutter and/or the like.

The catheters may include a monorail delivery system to assist in positioning the cutter at the target site. For example, the tip of the catheter can include lunen(s) that are sized to receive a conventional guidewire (typically 0.014" diameter) or any other suitable guidewire (e.g., having diameters between 0.018" and 0.032") and the flexible proximal portion of the catheter body can include a short lumen (e.g., about 12 centimeters in length). Such a configuration moves the guidewire out of the rigid portion so as to not interfere with the debulking assembly.

In other embodiments, however, the guidewire lumen may be disposed within or outside the flexible proximal portion of the catheter body and run a longer or shorter length, and in fact may run the entire length of the flexible portion of the catheter body. The guidewire can be disposed within lumen on the flexible portion of the catheter body and exit the lumen at a point proximal to the rigid portion of the catheter. The guidewire can then enter a proximal opening in the tip lumen and exit a distal opening in the tip lumen. In some embodiments, the catheter has a distal guidewire lumen on its flexible distal tip and a proximal guidewire lumen on its flexible body. For example, in some embodiments the distal lumen may have a length of between about 2.0 cm and about 3.0 cm and the proximal lumen may have a length of between about 10 cm and about 14 cm. In yet further embodiments, a distal tip guidewire lumen may be configured to telescope within a proximal guidewire lumen, or vice versa. A telescoping guidewire lumen may enhance performance of the catheter by preventing a guidewire from being exposed within a body lumen.

Any of a wide variety of conventional radiopaque markers, imaging devices, and/or transducers may be used. In exemplary embodiments, the catheters of the present invention can include a radiopaque distal portion and/or radiopaque markers disposed on a distal portion of the catheter body, such as proximal and distal of the cutting window, on the cam or ramp, so as to allow the user to track the position of the cutter, or the like. The catheters will also be particularly useful with ultrasonic transducers, such as an IVUS, of a type which may be deployed linearly within the catheter body or circumferentially on the debulking assembly. Linear deployment will allow viewing along a discrete length of the catheter axis, preferably adjacent to the cutting point, usually over a length in the range from 1 mm to 30 mm, preferably 2 mm to 10 mm. Circumferentially deployed phased arrays may subtend a viewing arc in the range from 5° to 360°, usually from 180° to 360°. For imaging transducers located on cutting blades within a housing or second cutting element, the field of imaging will generally be limited by the dimensions of the aperture. In some cases, however, it might be possible to fabricate all or a portion of the cutter blade/housing out of an ultrasonically translucent material. A more complete description of suitable imaging catheters are described more fully in U.S. Patent Application Serial No. 09/378,224, filed August 19,1999, and entitled "Atherectomy Catheter with Aligned Imager," now U.S. Patent No., 6,299,622 Bl. In addition to ultrasonic array transducers, the imaging devices of the present invention may comprise optical coherence tomography devices, such as described in U.S. Patent No. 5,491,524, as well as Huang et al. (1991) Science 254:1178-1181; Brezinski et al. (1997) Heart 77:397-403; and Brezinski et al (1996) Circulation 93:1206-1213.

### Kits

There is also described a kit comprising a catheter for removing vascular tissue from a vascular lumen of a patient, a device for removing a body sample such as blood, lymph, sweat, tears, urine, sputum, stool, or nonvascular tissue from the patient, and a container for both. The device for removing vascular tissue can be, for example, a FoxHollow Technologies atherectomy device or similar devices that remove vascular tissue. The device for removing blood or lymph or other body sample from the patient can be, e.g., a needle and syringe for extracting blood or lymph or other typical collection and/or removal devise, including a test tube, jar, microscope slide, solid phase antigen or antibody capture medium. A catheter or other percutaneous device can remove non-vascular tissue such as adipose or proliferating tissue, for example. The container for holding both the tissue removal device and the device for removing blood or lymph from the patient for marker analysis can be a box or other suitable container with an ability to close the items together for packaging, shipping, and storing. The kit can further comprise a device for measuring a marker in the removed blood, lymph, or vascular or nonvascular tissue, such as a device capable of analyzing small amounts of fluid or tissue for markers. Such devices include dipsticks, multiwell plates, slides, etc

There is also described a kit having a device (such as a catheter) for removing vascular tissue from the vascular lumen of a patient, and a device for measuring a marker from a patient, and a container for both of these devices. The device for measuring the marker can be, for example, one that provides contact between a small amount of blood, lymph, or tissue and a device for making a marker analysis. Procedures for analyzing markers are standard in the art.

### Methods of Modulating a Pathway

A method of modulating a pathway of molecular events in a patient having vascular disease can be effected by first identifying a representative event in the pathway, such as, for example, binding of two molecules, the presence or absence of a marker molecule, increase RNA expression, increase DNA expression, inflammation, infection, development of vascular disease (evidenced, for example, by a symptom in the patient that would indicate vascular disease, such as, e.g., reduced blood flow to the heart or through the vasculature), transcriptional activity, ligand binding, cell signaling, tissue proliferation in a vascular lumen, or an altered body chemistry (as evidenced, e.g., by a change in markers in the patient, or a change in other such indicia of a changed physiology in the patient, such as blood pressure, temperature, stamina, pain, or other such indicia). A baseline value for the representative event will be determined before the atherectomy procedure. The nature of the baseline value will depend on the representative event, so that each event will have its own characteristic value, and one or more ways to determine that value with standard assays, or measurement devices. The values will be selected from a value determined from both a population of otherwise healthy individuals not having appreciable vascular disease, and also from a value for this event specific to the patient having vascular disease, before treatment by removing vascular tissue. After treatment by removing vascular tissue, the value of the event can be measured again, to compare it to a baseline value. The baseline value will be that value in the patient having vascular disease, taken before the tissue removal. The baseline value can be compared to the values of a population of normal healthy patients in order to establish a relative condition in the patient being treated.

### Methods of Modifying a Marker

A method of modifying a marker in a patient having vascular disease can be accomplished by identifying the marker, and then removing sufficient vascular tissue to effect a modification of the marker. Here, the modification is not of the level of the marker, but of the character of the marker. For example, the modification can be a conformational change, a structural change, an addition of a moiety (e.g., an acetyl group, a phosphate group, a methyl group), a loss of a moiety, a change in the marker's activity, an increase in binding activity, or a decrease in binding activity. Any modification possible in any marker is contemplated, for example, a change in post-translational modification of an expressed protein, or a change in a cell activity, and other known changes possible in marker molecules of all types. The markers that are modified can be, for example, any of the markers listed or discussed herein. That the marker has actually been modified can be confirmed after removal of the vascular tissue, by removing, e.g., blood, lymph, or tissue (vascular or non-vascular) in order to test for the marker modification. Testing for the marker modification can be accomplished by standard means, as appropriate for the particular modification, in all cases.

The importance of modifying a marker can be that by changing a marker's character, its activity is also changed, and where treatment for vascular disease is the goal, a change in a marker's activity can represent an improvement of the patient's condition overall.

### Time Periods for Second Testing of Marker

The method can also further comprise measuring a level of the marker, or the character of the marker, after a pre-determined period of time after removal of vascular tissue in order to determine if more tissue should be removed to maintain a target level or character of a of marker. This step is distinct from measuring the marker level or character just after removal of the tissue in order to determine that sufficient tissue has been removed to effect a modulation in the marker. Measuring a marker level or character a second and optionally more times after removal of vascular tissue provides the opportunity to continue to keep the patient's marker levels and or characteristics within the target range. Thus, within a predetermined period of time (e.g., a pre-determined period of time such as approximately 1 minute, 1 hour, 4 hours, 6 hours, 12 hours, 1 day, 1 week, 1 month, 3 months, 6 months, 1 year, 2 years, 3 years, 4 years, 5 years, or 10 years) the marker level or character is measured again, and if it has fallen away from the target range, more vascular tissue is removed in order to bring the marker levels or characteristics back into normal range for the patient. The process can be repeated as part of an ongoing treatment of the patient.

### Markers

Particularly, it is of great advantage to reduce levels of markers of inflammation in patients having vascular disease. The markers that are decreased after a procedure comprising removal of vascular tissue would be those markers the increase of which indicates disease, and the markers that are increased after removal of vascular tissue are those markers that are more prevalent (or prevalent at higher levels) in patients not having vascular disease (or those patients having mild vascular disease) in comparison to sick patients. Thus, while markers of patients having vascular disease can be reduced by a procedure comprising removal of vascular tissue, (i.e. particularly markers of inflammation), many markers may be modulated (i.e. increased or decreased) depending on the nature of the marker and what the marker indicates about the condition of the patient.

The marker can be, for example, a marker for inflammation known as C reactive protein (CRP).

TABLE. 1 provides markers that can be measured but which are not a part of the invention. Markers that may also be used for the invention include the following protein or polypeptide markers or their corresponding encoding nucleic acid molecules (a few of which have been mentioned earlier): C reactive protein (CRP), interleukin-6 (IL-6), vascular endothelial growth factor, platelet-derived growth factor (PDGF), PDGF receptor, basis fibroblast growth factor (bFGF) This list is not intended to be exhaustive, but rather exemplary of the ongoing or repeated monitoring as described earlier may be conducted with the patient with any of these markers.

More than one marker may be measured, if appropriate and useful to understanding the condition of the patient. For example, both an inflammation marker and a marker indicating another aspect of a vascular condition can be measured, or two inflammation markers can be measured, for example. Each marker can be separately assessed for its modulation in response to the removal of vascular tissue. Two or several or a plurality of markers may be measured to provide useful information to determine the patient's condition after removal of the vascular tissue, and also to monitor the patient for determinations of future treatment, for example, to determine when it would be appropriate for a second or subsequent procedure to remove more vascular tissue. The marker may be an antibody specific for an antigen of interest.

In certain embodiments of the present invention the vascular tissue collected from the vascular lumen can be analyzed by standard well known methods for the presence of DNA, RNA, or protein markers comprising a whole host of possible tissue markers. Some exemplary tissue markers that can be analyzed from vascular tissue removed from the patient comprise, for example, smooth muscle proliferative promoters such as platelet-derived growth factor (PDGF) and PDGF receptor, basic fibroblast growth factor (FGF) vascular endothelial growth factor (VEGF), vascular cell adhesion molecule-1 (VCAM-1), interleukin 6 (IL-6), chemokines and chemokine receptors, inflammation markers (C-reactive protein),interleukins, interleukin 6 (IL-6), growth factors (platelet-derived growth factor (PDGF), basic fibroblast growth factor (FGF). Analysis of the excised tissue by any of the above tests can be used for diagnosis of a condition in a patient, design a treatment directive or protocol for a subject, monitor progress of a treatment regimen, or if tests from a number of individuals are compared, the information can be used in a multi-patient analysis, such as a vascular disease population study. Any of the tissue markers listed herein may also be sought in the circulating blood or lymph provided there is a way to measure the level of the particular marker for any corresponding form found in circulating blood or lymph of the patient.

### EXAMPLE

### EXAMPLE 1:

A patient is selected for an atherectomy procedure because some regions in his peripheral vasculature are identified by sonographic imaging as containing possible atherosclerotic tissue. Catheters for entry into the peripheral vasculature are prepared. An aliquot of the patient's blood is withdrawn and measurements are made for the presence of the inflammatory marker CRP, the marker LPPLA2, oxidized LDL, lipids, selectin, and lipopolysaccharide (LPS). The measurements for each marker are recorded. The patient then undergoes an atherectomy procedure during which approximately 200 grams total of atherosclerotic tissue is removed from the vasculature of both legs. The tissue itself is also analyzed and found to contain plaque, fibrous tissue, lipid, some vulnerable plaque, and inflamed tissue. The tissue is then analyzed for markers including DNA, RNA, and protein markers for PDGF, PDGF receptor, FGF, VEGF, VCAM-1, and IL-6. Blood is drawn from the patient within one hour of the atherectomy procedure and 3 days after the procedure and both aliquots are tested for the markers that were originally tested before the atherectomy procedure. In a comparison with the original measurements, the circulating level of CRP was returned to within a range of that for a person not having a serious atherosclerotic condition. The other circulating markers also tested were lowered. It was determined that sufficient vascular tissue had been removed from the patient in order to effect an improvement in the patient's blood chemistry and cardiac physiology by interpretation of the markers that were tested. It was recommended that the patient's markers be tested within 1 year of the operation, and that should the markers have returned to their original pre-surgical level, the patient would be considered for another atherectomy procedure to reduce them back to healthy levels.

Although the foregoing invention has been described in detail for purposes of clarity of understanding, it will be obvious that certain modifications may be practiced within the scope of the appended claims.

**TABLE 1**

| | |
|---|---|
| AA775616 | osteopontin |
| AA682386 | oxidised low density lipoprotein (lectin-like) receptor 1 |
| AA969504 | interferon, gamma |
| AA102526 | interleukin 8 |
| BU631490 | tissue inhibitor of metalloproteinase 2 |
| NM_002356 | myristoylated alanine-rich protein kinase C substrate |
| NM_000930 | plasminogen activator, tissue |
| NM_002117 | major histocompatibility complex, class I, C |
| AI129421 | interleukin 18 (interferon-gamma-inducing factor) |
| W51794 | matrix metalloproteinase 3 (stromelysin 1, progelatinase) |
| AA143201 | matrix metalloproteinase 1 (interstitial collagenase) |
| N94616 | laminin, alpha 4 |
| NM_021999 | integral membrane protein 2B |
| NM_000584 | interleukin 8 |
| NM_002510 | glycoprotein (transmembrane) nmb |
| N53447 | integral membrane protein 2A |
| NM_002659 | plasminogen activator, urokinase receptor |
| AL133111 | SH3-domain binding protein 5 (BTK-associated) |
| NM_147780 | cathepsin B |
| W46577 | endothelial cell-specific molecule 1 |
| AA857496 | matrix metalloproteinase 10 (stromelysin 2) |
| NM_005502 | ATP-binding cassette, sub-family A (ABC1), member 1 |
| AI342012 | macrophage scavenger receptor 1 |
| AA490846 | integrin, alpha 4 (antigen CD49D, alpha 4 subunit of VLA-4 receptor) |
| AA454999 | hypothetical protein FLJ 10111 . |
| AK093984 | hypothetical protein MGC5618 |
| AA666269 | integrin, beta 3 (platelet glycoprotein IIIa, antigen CD61) |
| NM_005625 | syndecan binding protein (syntenin) |
| BC014989 | phospholipid scramblase 3 |
| AI279830 | protein phosphatase 1, regulatory (inhibitor) subunit 16B |
| AA936768 | interleukin 1, alpha |
| NM_001920 | decorin |
| AK055130 | calmodulin 2 (phosphorylase kinase, delta) |
| NM_016497 | mitochondrial ribosomal protein L51 |
| AA451863 | CD4 antigen (p55) |
| NM_058197 | cyclin-dependent kinase inhibitor 2A |
| R10284 | hyaluronan-mediated motility receptor (RHAMM) |
| AI309439 | integrin, alpha M (complement component receptor 3, alpha) |
| AI334914 | integrin, alpha 2b |
| AF001893 | multiple endocrine neoplasia I |
| N36136 | endomucin-2 |
| AW772163 | hypothetical protein FLJ20401 |
| NM_001964 | early growth response 1 |
| AA454668 | prostaglandin-endoperoxide synthase 1 |
| NM_004530 | matrix metalloproteinase 2 |
| AK027663 | stanniocalcin 2 |
| AA057204 | interleukin 2 receptor, beta |
| NM_001444 | fatty acid binding protein 5 (psoriasis-associated) |
| AA873792 | small inducible cytokine A5 (RANTES) |
| interleukin 1, alpha | |
| NM_000600 | interleukin 6 (interferon, beta 2) |
| N98591 | interleukin 6 (interferon, beta 2) |
| AA156031 | metallothionein 2A |
| NM_001235 | serine (or cysteine),proteinase inhibitor, clade H |
| BF131637 | metallothionein 2A |
| NM_006216 | serine (or cysteine) proteinase inhibitor, clade E |
| NM_001552 | insulin-like growth factor binding protein 4 |
| NM_004530 | matrix metalloproteinase 2 |
| NM_000088 | collagen, type I, alpha 1 |
| NM_023009 | MARCKS-like protein |
| NM_003670 | basic helix-loop-helix domain containing, class B, 2 |
| T80495 | Hs. clone 24707 mRNA sequence |
| NM_002993 | chemokine C-X-C motif, granulocyte chemotactic protein 2 |
| NM_006756 | transcription elongation factor A (SII), 1 |
| AI983239 | Hs. cDNA FLJ32163 fis, clone PLACE6000371 |
| NM_005110 | glutamine-fructose-6-phosphate transaminase 2 |
| NM_000584 | interleukin 8 |
| AK092836 | Homo sapiens cDNA FLJ35517 fis, clone SPLEN2000698 NM_000104 cytochrome P450, subfamily I (dioxin-inducible), peptide NM_004966 heterogeneous nuclear ribonucleoprotein F |
| AK025599 | mannosidase, alpha, class 1A, member 1 |
| NM_002923 | regulator of G-protein signalling 2, 24kDa |
| AW005755 | macrophage migration inhibitory factor |
| AA873792 | small inducible cytokine A5 (RANTES) |
| U72621 | pleiomorphic adenoma gene-like 1 |
| NM_000358 | transforming growth factor, beta-induced, 68kDa |
| AK054688 | Homo sapiens cDNA FLJ30126 fis, clone BRACE1000114 |
| BC007583 | Homo sapiens, clone MGC:15572 IMAGE:3140342 |
| NM_000089 | collagen, type I, alpha 2 |
| NM_004404 | neural precursor cell expressed, developmental regulated 5 |
| NM_078467 | cyclin-dependent kinase inhibitor 1A (p21, Cip1) |
| U97105 | Homo sapiens N2A3 mRNA, complete cds |
| AI356451 | CD19 antigen |
| BF732465 | tissue inhibitor of metalloproteinase 2 |
| NM_001554 | cysteine-rich, angiogenic inducer, 61 |
| BQ890604 | Homo sapiens URB mRNA, complete cds |
| NM_002631 | phosphogluconate dehydrogenase |
| N94503 | pregnancy-associated plasma protein A |
| NM_001710 | B-factor, properdin |
| interleukin 8 | |
| N98591 | interleukin 6 (interferon, beta 2) |
| A936768 | interleukin 1, alpha |
| BM803108 | ESTs |
| NM_000600 | interleukin 6 (interferon, beta 2) |
| AI359876 | EST |
| AA156031 | metallothionein 2A |
| BF131637 | metallothionein 2A |
| NM_003670 | basic helix-loop-helix domain, class B, 2 |
| NM_001235 | serine (or cysteine) proteinase inhibitor, clade H |
| NM_004530 | matrix metalloproteinase 2 |
| NM_002982 | monocyte chemotactic protein 1 |
| NM_002631 | phosphogluconate dehydrogenase |
| NM_078467 | cyclin-dependent kinase inhibitor 1A (p21, Cipl) |
| NM_152862 | actin related protein 2/3 complex, subunit 2 |
| NM_002923 | regulator of G-protein signalling 2, 24kDa |
| AI983239 | Hs. cDNA FLJ32163 fis, clone PLACE6000371 |
| NM_005415 | solute carrier family 20, member 1 |
| AW772163 | hypothetical protein FLJ20401 |
| R21535 | Hs. cDNA FLJ11724 fis, clone HEMBA1005331 |
| NM_005110 | glutamine-fructose-6-phosphate transaminase 2 |
| AK092836 | cDNA FLJ35517 fis, clone SPLEN2000698 |
| NM_006216 | serine (or cysteine) proteinase inhibitor, clade E |
| | |
| interleukin 6 (interferon, beta 2) | |
| N98591 | interleukin 6 (interferon, beta 2) |
| NM_005746 | pre-B-cell colony-enhancing factor |
| NM_002852 | pentaxin-related gene, rapidly induced by IL-1 beta |
| N92901 | fatty acid binding protein 4, adipocyte |
| NM_005110 | glutamine-fructose-6-phosphate transaminase 2 |
| AK094728 | cDNA FLJ37409 fis, similar to COMPLEMENT C3 |
| NM_004000 | chitinase 3-like 2 |
| NM_002923 | regulator of G-protein signalling 2, 24kDa |
| T80495 | Hs. clone 24707 mRNA sequence |
| AA936768 | interleukin 1, alpha |
| NM_145791 | microsomal glutathione S-transferase 1 |
| NM_006169 | nicotinamide N-methyltransferase |
| AW007736 | UDP-glucose ceramide glucosyltransferase |
| NM_005420 | sulfotransferase, estrogen-preferring |
| NM_003670 | basic helix-loop-helix domain containing, class B, 2 |
| AA425102 | monocyte chemotactic protein 1 |
| NM_003254 | tissue inhibitor of metalloproteinase 1 |
| BF131637 | metallothionein 2A |
| NM_000104 | cytochrome P450, subfamily I (dioxin-inducible) |
| NM_001733 | complement component 1, r subcomponent |
| NM_032849 | hypothetical protein FLJ14834 |
| M_005328 | hyaluronan synthase 2 |
| NM_002009 | fibroblast growth factor 7 (keratinocyte growth factor) |
| NM_002615 | serine (or cysteine) proteinase inhibitor, clade F |
| NM_002658 | plasminogen activator, urokinase |
| NM_033439 | DVS27-related protein |
| AA381343 | interleukin 6 (interferon, beta 2) |
| AW780123 | ribosomal protein S26 |
| M14219 | chondroitin/dermatan sulfate proteoglycan (PG40) core |
| AF495759 | Homo sapiens unknown mRNA |
| NM_001679 | ATPase, Na+/K+ transporting, beta 3 polypeptide |
| NM_001029 | ribosomal protein S26 |
| NM_002074 | guanine nucleotide binding protein, beta polypeptide 1 |
| NM_001552 | insulin-like growth factor binding protein 4 |
| AF208043 | interferon, gamma-inducible protein 16 |
| AI268937 | monocyte chemotactic protein 2 |
| AA040170 | monocyte chemotactic protein 3 |
| AW131311 | EST |
| NM_005415 | solute carrier family 20 (phosphate transporter), member 1 |
| NM_006988 | a disintegrin-like and metalloprotease (reprolysin type) |
| NM_006307 | sushi-repeat-containing protein, X chromosome |
| NM_000584 | interleukin 8 |
| D31887 | KIAA0062 protein |
| NM_002229 | jun B proto-oncogene |
| NM_002982 | monocyte chemotactic protein 1 |
| serine (or cysteine) proteinase inhibitor, clade F | |
| A094728 | Homo sapiens cDNA FLJ37409 fis, clone-BRAMY2028516 |
| NM_001552 | insulin-like growth factor binding protein 4 |
| N92901 | fatty acid binding protein 4, adipocyte |
| N98591 | interleukin 6 (interferon, beta 2) |
| NM_000104 | cytochrome P450, subfamily I (dioxin-inducible) |
| NM_006756 | transcription elongation factor A (SII), 1 |
| NM_000600 | interleukin 6 (interferon, beta 2) |
| AF506819 | Homo sapiens URB mRNA, complete cds |
| NM_145791 | microsomal glutathione S-transferase 1 |
| N39161 | CD36 antigen (thrombospondin receptor) |
| M14219 | Human chondroitin sulfate proteoglycan core protein |
| NM_031476 | hypothetical protein DKFZp434B044 |
| NM_000186 | H factor 1 (complement) |
| NM_003254 | tissue inhibitor of metalloproteinase 1 |
| N98591 | interleukin 6 (interferon, beta 2) |
| AJ318805 | ESTs, Weakly similar to hypothetical protein FLJ20378 |
| A284954 | colony stimulating factor 1 receptor |
| NM_002923 | regulator of G-protein signalling 2, 24kDa |
| NM_001920 | decorin |
| BI830199 | likely ortholog of mouse Urb |
| AA451863 | CD4 antigen (p55) |
| AA464526 | interleukin 1 receptor, type I |
| AW192258 | sprouty homolog 4 (Drosophila) |
| N68859 | intercellular adhesion molecule 1 (CD54) |
| BC007552 | Homo sapiens, clone MGC:15473 IMAGE:2967168, mRNA |
| NM_001733 | complement component 1, r subcomponent |
| NM_006288 | Thy-1 cell surface antigen |
| NM_000201 | intercellular adhesion molecule 1 (CD54) |
| R22412 | platelet/endothelial cell adhesion molecule (CD31 antigen) |
| NM_013417 | isoleucine-tRNA synthetase |
| NM_004000 | chitinase 3-like 2 |
| R70506 | growth factor receptor-bound protein 2 |
| NM_030781 | collectin sub-family member 12 |
| NM_001710 | B-factor; properdin |
| NM_006216 | serine (or cysteine) proteinase inhibitor, clade E |
| NM_005110 | glutamine-fructose-6-phosphate transaminase 2 |
| AF506819 | Homo sapiens URB mRNA, complete cds |
| NM_002074 | guanine nucleotide binding protein, beta polypeptide 1 |
| H26022 | fractalkine, inducible cytokine subfamily D (Cys-X3-Cys) |
| AK092836 | Homo sapiens cDNA FLJ35517 fis, clone SPLEN2000698 |
| BQ890604 | Homo sapiens URB mRNA, complete cds |
| AA057204 | interleukin 2 receptor, beta |
| AI524093 | myosin, heavy polypeptide 11, smooth muscle |
| AI655374 | stromal cell-derived factor 1 |

## Claims

1. An *ex vivo* method of monitoring the success of atherectomy, the atherectomy comprising removal of diseased vascular tissue from a vascular lumen of a patient, wherein the method comprises testing a sample obtained from a patient prior to atherectomy and testing a sample obtained from the patient after atherectomy for the level of a marker, wherein a decrease in the level of a marker selected from C reactive protein (CRP), PDGF, PDGF receptor, FGF, VEGF, VCAM-1 and IL-6 indicates success of the atherectomy.

2. A method as claimed in claim 1, wherein the diseased vascular tissue comprises tissue selected from arterial plaque, vulnerable plaque, inflamed tissue, arterial tissue, calcified tissue, thrombotic tissue, lipid-rich tissue, foam cell tissue, macrophage-rich tissue, hypocellular tissue, fibrotic tissue, and hypercellular tissue.

3. A method as claimed in claim 1 or claim 2, wherein the diseased vascular tissue is removed from a vessel selected from a peripheral artery, a coronary artery, and a carotid artery.

4. A method as claimed in any one of claims 1 to 3, wherein said first and second samples are selected from lymph, saliva, sputum, urine, stool, blood, sweat and tears, and preferably blood.

5. A method as claimed in any one of claims 1 to 4, wherein the marker is CRP.

6. A method as claimed in any one of claims 1 to 4, wherein the marker is selected from PDGF, PDGF receptor, FGF, VEGF, VCAM-I, and IL-6.

7. A method according to any one of claims 1 to 6, wherein the second level is determined between 12 hours and 14 days after removal of said diseased vascular tissue from said patient.

8. A method according to anyone of claims 1 to 6, wherein the second level is determined between 6 months and 2 years after removal of said diseased vascular tissue from said patient.

9. A method according to any one of claims 1 to 6, wherein the second level is determined between 1 year and 5 years of said removing.

## Patentansprüche

1. Ex-vivo-Verfahren zur Überwachung des Erfolgs einer Atherektomie, wobei die Atherektomie die Entfernung von krankem Gefäßgewebe aus einem Gefäßlumen eines Patienten umfasst, worin das Verfahren die Untersuchung einer von einem Patienten vor der Atherektomie erhaltenen Probe und die Untersuchung einer von dem Patienten nach der Atherektomie erhaltenen Probe auf eine Konzentration eines Markers umfasst, worin eine Verringerung der Konzentration eines Markers, der unter C-reaktives Protein (CRP), PDGF, PDGF-Rezeptor, FGF, VEGF, VCAM-1 und IL-6 ausgewählt ist, auf den Erfolg der Atherektomie hinweist.

2. Verfahren nach Anspruch 1, worin das kranke Gefäßgewebe Gewebe umfasst, das unter arterieller Plaque, vulnerabler Plaque, entzündetem Gewebe, arteriellem Gewebe, verkalktem Gewebe, thrombotischem Gewebe, lipidreichem Gewebe, Schaumzellgewebe, makrophagenreichem Gewebe, hypozellulärem Gewebe, fibrotischem Gewebe und hyperzellulärem Gewebe ausgewählt ist.

3. Verfahren nach Anspruch 1 oder Anspruch 2, worin das kranke Gefäßgewebe aus einem Gefäß entfernt wird, das unter einer peripheren Arterie, einer Koronararterie und einer Karotis ausgewählt ist.

4. Verfahren nach einem der Ansprüche 1 bis 3, worin die ersten und zweiten Proben unter Lymphe, Speichel, Sputum, Urin, Stuhl, Blut, Schweiß und Tränen ausgewählt sind und vorzugsweise Blut sind.

5. Verfahren nach einem der Ansprüche 1 bis 4, worin der Marker CRP ist.

6. Verfahren nach einem der Ansprüche 1 bis 4, worin der Marker unter PDGF, PDGF-Rezeptor- FGF, VEGF, VCAM-1 und IL-6 ausgewählt ist.

7. Verfahren nach einem der Ansprüche 1 bis 6, worin die zweite Konzentration 12 Stunden bis 14 Tage nach Entfernung des kranken Gefäßgewebes aus dem Patienten bestimmt wird.

8. Verfahren nach einem der Ansprüche 1 bis 6, worin die zweite Konzentration 6 Monate bis 2 Jahre nach Entfernung des kranken Gefäßgewebes aus dem Patienten bestimmt wird.

9. Verfahren nach einem der Ansprüche 1 bis 6, worin die zweite Konzentration 1 Jahr bis 5 Jahre nach der Entfernung bestimmt wird.

## Revendications

1. Procédé *ex vivo* de surveillance de la réussite d'une athérectomie, l'athérectomie comprenant le retrait du tissu vasculaire malade d'un lumen vasculaire chez un patient, dans lequel le procédé comprend l'étape consistant à tester un échantillon prélevé sur un patient avant l'athérectomie et à tester un échantillon prélevé sur le patient après l'athérectomie pour rechercher le taux d'un marqueur, où une diminution du taux d'un marqueur choisi parmi la protéine C réactive (CRP), le PDGF, le récepteur du PDGF, le FGF, le VEGF, la VCAM-1 et l'IL-6 indique la réussite de l'athérectomie.

2. Procédé selon la revendication 1, dans lequel le tissu vasculaire malade comprend du tissu choisi parmi la plaque artérielle, la plaque vulnérable, le tissu enflammé, le tissu artériel, le tissu calcifié, le tissu thrombotique, le tissu riche en lipide, le tissu de cellules spumeuses, le tissu riche en macrophages, le tissu hypocellulaire, le tissu fibrotique, et le tissu hypercellulaire.

3. Procédé selon la revendication 1 ou la revendication 2, dans lequel le tissu vasculaire malade est retiré d'un vaisseau choisi parmi une artère périphérique, une artère coronaire, et une artère carotidienne.

4. Procédé selon l'une quelconque des revendications 1 à 3, dans lequel lesdits premier et second échantillons sont choisis parmi la lymphe, la salive, le crachat, l'urine, les fèces, le sang, la sueur et les larmes, et de préférence le sang.

5. Procédé selon l'une quelconque des revendications 1 à 4, dans lequel le marqueur est la CRP.

6. Procédé selon l'une quelconque des revendications 1 à 4, dans lequel le marqueur est choisi parmi le PDGF, le récepteur du PDGF, le FGF, le VEGF, la VCAM-1 et l'IL-6.

7. Procédé selon l'une quelconque des revendications 1 à 6, dans lequel le second taux est déterminé entre 12 heures et 14 jours après le retrait dudit tissu vasculaire malade chez ledit patient.

8. Procédé selon l'une quelconque des revendications 1 à 6, dans lequel le second taux est déterminé entre 6 mois et 2 ans après le retrait dudit tissu vasculaire malade chez ledit patient.

9. Procédé selon l'une quelconque des revendications 1 à 6, dans lequel le second taux est déterminé entre 1 an et 5 ans après ledit retrait.
